Europäisches Patentamt

European Patent Office

Office européen des brevets

(19)

(11) Veröffentlichungsnummer : **0 420 368 B1**

# (12) EUROPÄISCHE PATENTSCHRIFT

(45) Veröffentlichungstag der Patentschrift :
**04.08.93 Patentblatt 93/31**

(51) Int. Cl.[5] : **A61K 31/60,** // (A61K31/60,
31:557)

(21) Anmeldenummer : **90250248.3**

(22) Anmeldetag : **01.10.90**

(54) **Kombinationspräparat mit antithrombotischer Wirkung.**

(30) Priorität : **29.09.89 DE 3933027**

(43) Veröffentlichungstag der Anmeldung :
**03.04.91 Patentblatt 91/14**

(45) Bekanntmachung des Hinweises auf die
Patenterteilung :
**04.08.93 Patentblatt 93/31**

(84) Benannte Vertragsstaaten :
**AT BE CH DE DK ES FR GB GR IT LI LU NL SE**

(56) Entgegenhaltungen :
**EP-A- 0 208 962
PROSTAGLANDINS, Band 36, Nr. 6, Dezember
1988, Seiten751-760
THROMBOSIS AND HAEMOSTASIS, Band 61,
Nr. 2, 25 April 1989, Seiten 286-288.
V.BERTELE et al.: "The inhibitory effect of
aspirin on fibrinolysis is reversed by iloprost,
a prostacyclin analogue"**

(73) Patentinhaber : **SCHERING
AKTIENGESELLSCHAFT Berlin und
Bergkamen
Müllerstrasse 170/178 Postfach 65 03 11
W-1000 Berlin 65 (DE)**

(72) Erfinder : **Witt, Werner
Motzstrasse 25
W-1000 Berlin 30 (DE)**
Erfinder : **Baldus, Berthold
Joachim-Friedrich-Strasse 13
W-1000 Berlin 31 (DE)**
Erfinder : **Müller, Bernd
Grünlandweg 12
W-1000 Berlin 26 (DE)**
Erfinder : **Stürzebecher, Claus-Steffen
Sponholzstrasse 63
W-1000 Berlin 41 (DE)**
Erfinder : **Skuballa, Werner
Mattersburger Weg 12
W-1000 Berlin 28 (DE)**

**Beschreibung**

Die Erfindung betrifft ein Kombinationserzeugnis zur Hemmung der Thrombozytenfunktion und zur Thrombosebehandlung, enthaltend die Carbacyclinderivate Cicaprost oder Eptaloprost und Acetylsalicylsäure (ASS).

Bei der arteriellen und mit gewissen Einschränkungen auch bei der venösen Thrombogenese spielt die Aktivierung der Thrombozyten mit nachfolgender Aggregation eine zentrale Rolle. Diese Plättchenaktivierung wird durch Stimuli ausgelöst, die bei einer Gefäßwandschädigung und Gerinnungsaktivierung auftreten können, wie z.B. durch Thrombin, Kollagen, Adenosindiphosphat, Adrenalin oder durch den plättchenaktivierenden Faktor (PAF). Diese Stimuli lösen neben der Aggregation gleichzeitig die Freisetzung von Stoffen aus Thrombozyten aus, die in sogenannten Granula der Thrombozyten gespeichert sind. Einige dieser Stoffe. wie z.B. Plasminogenaktivator-Inhibitor-1 (PAI-1) oder 'platelet-derived growth factor' (PDGF), sind wahrscheinlich von besonderer Bedeutung für die Pathogenese thrombotisch/atherosklerotischer Erkrankungen.

So ist PAI-1 ein potenter Inhibitor von Plasminogenaktivatoren wie Urokinase und t-PA und hemmt durch diese Eigenschaft die zur Wiederherstellung der Durchblutung nach thrombotischem Verschluß notwendige Fibrinolyse. PDGF ist ein Mitogen, das die Proliferation verschiedener Zelltypen (besonders glatter Muskelzellen) stimuliert. Aufgrund dieses Mechanismus wird dem PDGF eine Schlüsselrolle in der Atherogenese zugeschrieben. Die Hemmung nicht nur der Aggregation der Thrombozyten sondern auch der Freisetzung von pathogenen Stoffen wie PAI-1 und PDGF könnte einen wichtigen Beitrag zur Prophylaxe thromboembolischer und atherosklerotischer Erkrankungen leisten.

Die als Thrombozytenaggregationshemmer bekannte Acetylsalicylsäure ist nur ein schwacher Hemmstoff für die Thrombozytenfunktion und auch nur ein schwaches Antithrombotikum. Als Cyclooxygenasehemmstoff inhibiert Acetylsalicylsäure nur einen Aktivierungsweg der Thrombozyten, die Thromboxanbildung. Zusätzlich wird durch Acetylsalicylsäure im Gefäßendothel die Bildung von Prostacyclin, einem natürlichen Hemmstoff der Thrombozyten, inhibiert. Daher ist die therapeutische Wirkstärke von Acetylsalicylsäure für die Behandlung vieler thromboembolischer Krankheitsformen nicht ausreichend.

Mimetika des natürlichen Prostacyclins, wie die genannten Carbacylinderivate stehen seit kurzem für die klinische Anwendung in oraler Form zu Verfügung. Sie gelten als Hemmstoffe aller wesentlichen Aktivierungswege der Thrombozyten und starke Thrombozytenhemmer und Antithrombotika. Zusätzlich besitzen sie noch weitere Eigenschaften, so. z.B. die Fähigkeit zu Relaxation glatter Gefäßmuskulatur, die ihre therapeutische Wirkung bei bestimmten Krankheitsformen unterstützen. Bei höheren Dosierungen tritt das für die Stoffklasse der Carbacycline charakteristische Nebenwirkungsprofil in den Vordergrund, wodurch die anwendbare Höchstdosis limitiert und der vollen klinischen Ausnutzung der Thrombozytenfunktions-Hemmwirkung und der antithrombotischen Wirkung Grenzen gesetzt sind.

Es wurde nun überraschenderweise gefunden, daß die Kombinationspräparate aus Cicaprost und Acetylsalicylsäure und aus Eptaloprost und Acetylsalicylsäure in unterschiedlichen biologischen Systemen synergistisch wirken oder kooperative Effekte der Einzelwirkstoffe zeigen.

Beide Kombinationen übertreffen z.B. die antithrombotische Wirkstärke der Acetylsalicylsäure und erlauben eine erhebliche Dosisreduktion für den thrombozytenhemmenden Carbacyclinanteil im Vergleich zu equipotenten Dosierungen des allein verabreichten Carbacyclins (Cicaprost, Eptaloprost) um einen Faktor bis zu 10.

Damit sind die Kombinationen stärkere Thrombozytenhemmer und stärkere antithrombotische Mittel als Acetylsalicylsäure mit einer höheren Spezifität und therapeutischen Breite im Vergleich zu Cicaprost/Eptaloprost durch kooperative Verstärkung ihrer thrombozytären Wirkkomponente. Durch die magenprotektiven Eigenschaften der Carbacycline werden durch Acetylsalicylsäure hervorgerufene unerwünschte gastrointestinale Nebenwirkungen herabgesetzt.

Die Erfindung betrifft somit ein Kombinationspräparat aus Acetylsalicylsäure und Cicaprost oder Eptaloprost, wobei die Carbacycline auch in Form ihrer Additionssalze mit physiologisch verträglichen Basen oder deren Clathrate mit Cyclodextrinen eingesetzt werden können, sowie Arzneimittel mit den üblichen Hilfs- und Trägerstoffen zur Hemmung der Thrombozytenfunktion und zur Thrombosebehandlung.

Eptaloprost [(5E)-(16S)-13,14-Didehydro-1a, 1b-dihomo-16,20-dimethyl-3-oxa-18, 18,19,19-tetradehydro-6a-carbaprostaglandin-$I_2$] und sein β-Cyclodextrinclathrat können nach folgender Vorschrift hergestellt werden:

Zu einer Mischung aus 6,9 g 2-[(E)-(1S,5S,6S,7R)-7-(Dimethyl-tert.-butylsilyloxy)-6-[(3S,4S)-3-(dimethyl-tert.-butylsilyloxy)-4-methyl-nona-1,6-divinyl]bicyclo[3.3.0]octan-3-yliden]-äthan-1-ol [W. Skuballa, E. Schillinger, C.-S. Stürzebecher, H. Vorbrüggen, J. Medicinal Chemistry 29, 313(1986); hierin als Verbindung 15a beschrieben] und 11,5 g Trimethyl-ortho-4-brombutyrat fügt man 17,2 ml einer 50 %igen Natronlauge und 337 mg Tetrabutylammoniumhydrogensulfat und rührt 16 Stunden bei 22° C unter Argon. Anschließend verdünnt man bei Eiswasserkühlung mit 20 ml Wasser und säuert mit 10 %iger Citronensäurelösung auf pH 5 an. Man extrahiert dreimal mit je 300 ml Ether, wäscht die organische Phase einmal mit 200 ml Sole trocknet über Ma-

2

gnesiumsulfat und dampft im Vakuum ein. Nach Chromatographie des Rückstands an Kieselgel mit Hexan/Ether (8+2) erhält man 7,6 g (5E)-(16S)-13,14-Didehydro-1a, 1b-dihomo-16,20-dimethyl-3-oxa-18,18,19,19-tetra-dehydro-6a-carbaprostaglandin-$I_2$-methylester-11,15-bis-(dimethyl-tert.-butyl-ether) als farbloses Öl. IR(CHCl$_3$) : 2953, 2925, 2859, 2230, 1730, 1250, 838 cm$^{-1}$.

Zur Silyletherspaltung rührt man 7,25 g des vorstehend beschriebenen Bissilylethers 48 Stunden bei 24° C mit 600 ml einer Mischung aus Essigsäure/Wasser/Tetrahydrofuran (65+35+10). Man dampft anschließend im Vakuum ein und chromatographiert den Rückstand an Kieselgel. Mit Essigester/Hexan (3+2) erhält man 3,9 g (5E)-(16S)-13,14-Didehydro-1a,1b-dihomo-16,20-dimethyl-3-oxa-18,18,19,19-tetradehydro-6a-carba-prostaglandin-$I_2$-methylester als farbloses Öl.

IR(CHCl$_3$): 3400(breit). 2935, 2865, 2230, 1735 cm$^{-1}$.

Zur Verseifung rührt man eine Lösung aus 3,66 g des vorstehend hergestellten Methylesters in 35 ml Methanol mit 35 ml einer 0,5 molaren Natronlauge 30 Minuten bei 24° C unter Argon. Anschließend verdünnt man mit 20 ml Wasser, säuert mit einer 20 %igen Citronensäurelösung auf pH 2 an, extrahiert viermal mit je 100 ml Methylenchlorid, wäscht die organische Phase einmal mit 50 ml Sole, trocknet über Natriumsulfat und dampft im Vakuum ein. Den Rückstand chromatographiert man mit Essigester an Kieselgel. Dabei erhält man 3,4 g der Titelverbindung als farbloses Öl.

IR(CHCl$_3$): 3400(breit), 2962, 2940, 2865, 2230, 1722 cm$^{-1}$.

β-Cyclodextrinclathrat von (5E)-(16S)-13,14-Didehydro-1a,1b-dihomo-16,20-dimethyl-3-oxa-18,18,19, 19-tetra-dehydro-6a-carba-prostaglandin-$I_2$.

Man löst 41,75 g β-Cyclodextrin in 298 ml Wasser bei 80° C und tropft eine Lösung von 1,5 g (5E)-(16S)-13,14-Didehydro-1a,1b-dihomo-16,20-dimethyl-3-oxa-18,18,19,19-tetradehydro-6a-carba-prostaglandin-$I_2$ in 24 ml Ethanol innerhalb von 15 Minuten zu. Man rührt 4 Stunden bei 60° C und läßt dann über Nacht unter Rühren abkühlen. Der ausgefallene Feststoff wird abgesaugt, mit 50 ml einer Mischung aus Wasser-Ethanol (1:1) gewaschen und 24 Stunden bei 0,1 Torr und 25° C über Phosphorpentoxid getrocknet. Man erhält 38 g frei fließende Kristalle des β-Cyclodextrinclathrats des o.a. Carbacyclinanalogons. Der Gehalt an Carbacyclinanalogon im Clathrat wird durch Titration bestimmt und beträgt 3,3 %.

Cicaprost und sein β-Cyclodextrinclathrat können entsprechend den in der EP-A-0 119 949 und in der internationalen Offenlegungsschrift WO 87/05294 beschriebenen Verfahren hergestellt werden.

Zur Salzbildung sind anorganische und organische Basen geeignet, wie sie dem Fachmann zur Bildung physiologisch verträglicher Salze bekannt sind. Beispielsweise genannt seien Alkalihydroxide, wie Natrium- und Kaliumhydroxid, Erdalkalihydroxide wie Calciumhydroxid, Ammoniak, Amine, wie Äthanolamin, Diäthanolamin, Triäthanolamin, N-Methylglucamin, Morpholin, Tris-(hydroxymethyl)-methylamin usw. Bevorzugtes Cyclodextrin für die Clathratbildung ist das β-Cyclodextrin.

Acetylsalicylsäure und die genannten Carbacycline werden in Mengen eingesetzt, die unterhalb der sonst für die einzelnen Stoffe der Kombination üblichen Mengen liegen. Die entsprechend der Erfindung anzuwendende Menge hängt vom Umfang der Thrombose ab.

Die erfindungsgemäße Kombination wird vorzugsweise oral verabreicht. Acetylsalicylsäure und Carbacyclin stehen in einem Gewichtsverhältnis 1:5·10$^{-6}$ bis 1:1 zueinander, wobei eine Carbacyclin-Dosiseinheit 1-1000 μg und eine Acetylsalicylsäure-Dosiseinheit 1-500 mg beträgt.

## Beispiel 1

Humanes plättchenreiches Plasma (PRP) wird in einem Aggregometer bei 37°C inkubiert und zur Auslösung der Aggregation mit 3 ng/ml Kollagen versetzt. Cicaprost und ASS sowie Kombinationen beider Stoffe werden 1 Minute vor Zugabe des Aktivators Kollagen zu dem Ansatz pipettiert. Der Aggregationsverlauf wird im Aggregometer registriert. 10 Minuten nach Kollagen-Zugabe werden die Proben bei 3000 Upm abzentrifugiert und die Überstände mit einem 'enzyme linked immunosorbent assay' (ELISA) auf ihren Gehalt an Plasminogenaktivator-Inhibitor-1 (PAI-1) und mit einem 'radio-immuno-assay' (RIA) auf den Gehalt an 'platelet-derived growth factor' (PDGF) getestet.

Ergebnisse:

Die allein unwirksamen Konzentrationen von 10$^{-10}$ bzw. 10$^{-9}$ M Cicaprost führen in Kombination mit den ebenfalls un- bzw. schwach wirksamen Konzentrationen von 3x10$^{-5}$, 10$^{-4}$ und 3x10$^{-4}$ M ASS zu Thrombozytenaggregationshemmungen von 42%, 71% und 91% (Tabelle 1). Die Ausschüttung von PAI-1 wird bei gleichen Inhibitor-Konzentrationen zu 36%, 54% und 64%, die Freisetzung von PDGF zu 24%, 42% und 55% gehemmt. Die Wirkung der Cicaprost/ASS Kombinationen ist entsprechend der Definition von M.C.Berenbaum (Clin.exp.Immunol. 28:1-18, 1977) synergistisch.

**Tabelle 1**

Hemmung von Aggregation sowie PAI-1 und PDGF

Freisetzung an mit Kollagen stimulierten menschlichen Thrombozyten

in % der Kontrollreaktion (N = 6)

| | % INHIBITION | | |
| --- | --- | --- | --- |
| | Aggregation | PAI-1 | PDGF |
| Cicaprost $10^{-10}$M | 4 ± 3 | 0 ± 8 | 1 ± 2 |
| $10^{-9}$M | 0 ± 3 | 0 ± 12 | 3 ± 3 |
| $10^{-8}$M | 95 ± 4 | 72 ± 3 | 81 ± 3 |
| $10^{-7}$M | 95 ± 3 | 80 ± 5 | 81 ± 1 |
| ASS $3 \times 10^{-5}$M | 2 ± 1 | 6 ± 10 | 8 ± 2 |
| $10^{-4}$M | 9 ± 3 | 10 ± 8 | 26 ± 6 |
| $3 \times 10^{-4}$M | 30 ± 6 | 28 ± 4 | 39 ± 2 |
| $10^{-3}$M | 46 ± 6 | 27 ± 10 | 47 ± 4 |
| Cicaprost $10^{-10}$M + ASS $3 \times 10^{-5}$M | 42[a] ± 17 | 36 ± 14 | 24 ± 10 |
| Cicaprost $10^{-9}$M + ASS $10^{-4}$M | 71[a] ± 12 | 54[a] ± 9 | 42 ± 8 |
| Cicaprost $10^{-9}$M + ASS $3 \times 10^{-4}$M | 91[a] ± 2 | 64[a] ± 5 | 55[a] ± 6 |

[a] signifikant gegen ASS und Cicaprost Einzelgabe ( = 5%, Rangsummentest)

## Beispiel 2

An narkotisierten Meerschweinchen werden durch Injektion von Collagen (40 g/kg i.v.) reversible Thrombozytopenien erzeugt. Die Ursache dieser Thrombozytopenien ist eine Aggregatbildung der Collagen-stimulierten Blutplättchen und die Embolisation dieser Plättchenaggregate in die Lunge, wo diese dann steckenbleiben und so die beobachteten Abfälle der Plättchenzahl im Blut (Thrombozytopenien) auslösen. Die unter Kontrollbedingungen konstanten Thrombozytopenien (im Mittel 40 % Abfälle der Plättchenzahl vom jeweiligen Basalniveau) dienen als Maß der intravasalen Plättchenaggregation.

Ergebnisse

Die allein unwirksame Dosis von 1 ng/kg/min i.v. Cicaprost führt in Kombination mit der schwach hemmenden Dosis von 0,5 mg/kg i.v. ASS zu einer Thrombozytopenienhemmung von 34 %, signifikant gegen Kontrolle und die jeweilige Einzeleingabe der beiden Wirkstoffe (Tabelle 2). Dieser Effekt wird sonst nur durch eine 3fach höhere Dosis Cicaprost erreicht.

**Tabelle 2:** Hemmung der Collagen-induzierten Thrombozytopenie am Meerschweinchen (Mittelwerte ± SEM).

|  | N | % Thrombozytopenienhemmung |
|---|---|---|
| Kontrolle | 8 | $2 \pm 6$ |
| Cicaprost 1 ng/kg/min | 6 | $8 \pm 5^{n.s.}$ |
| 3 ng/kg/min | 6 | $36 \pm 4^{a}$ |
| ASS 0,5 mg/kg | 8 | $17 \pm 3^{a}$ |
| ASS 0,5 mg/kg + Cicaprost 1 ng/kg/min | 8 | $34 \pm 5^{a,b}$ |

[a] signifikant gegen Kontrolle (t-Test, p < 0,05)
[b] signifikant gegen ASS/Cicaprost (p < 0,01)
[n.s.] nicht signifikant

## Beispiel 3

An narkotisierten Meerschweinchen wird eine Mesenterialschlinge vorgelagert und mit temperierter Kochsalzlösung superfundiert. Unter intravitalmikroskopischer Kontrolle wird die Gefäßwand einer Mesenterialarteriole (Ø 20 - 50 m) durch eine Serie elektrischer Impulse geschädigt. Durch anschließende lokale Applikation aufsteigender Konzentrationen ADP (Adenosindiphosphat)-Lösung wird eine thrombogene ADP-Konzentration ermittelt, bei der sich ein okkludierender Plättchenthrombus an der vorgeschädigten Stelle der Arteriole bildet. Diese thrombogene ADP-Konzentration erzeugt gefäßspezifisch und reproduzierbar Thromben in der betreffenden Arteriole.

Ergebnisse

ASS 10 mg/kg i.v. verändert die thrombogene ADP-Konzentration nicht (Tabelle 3). Cicaprost 10 ng/kg/min i.v. ist ebenfalls in dieser Dosierung nicht signifikant wirksam. Die Kombination dieser an diesem Thrombosemodell unwirksamen Dosen Cicaprost und ASS führt zu einer signifikanten Steigerung der thrombogenen ADP-Konzentration um $1,27 + 0.54 \log M$ ($\hat{=}$ das 30,9-fache der Ausgangskonzentration). Diese antithrombotische Wirkung der Kombination kann durch ASS alleine nicht erreicht werden und durch Cicaprost-Einzelgabe nur in einer-10-fachen höheren Dosierung (100 ng/kg/min i.v.: Steigerung um $1.32 \pm 0.2 \log M$ ADP, N = 5).

**Tabelle 3:** Wirkung von Cicaprost, ASS und einer Kombination von Cicaprost + ASS auf die Entwicklung von okkludierenden Plättchenthromben an vorgeschädigten Mesenterialarteriolen des Meerschweinchens (Mittelwerte + SEM).

| | | N | Thrombogene ADP-Konzentrationen | |
| --- | --- | --- | --- | --- |
| | | | Ausgangswert [µM] | Steigerung [logµM] |
| Cicaprost | 10 ng/kg/min | 5 | $84.6 \pm 54.9$ | $+ 0.36^{n.s.} \pm 0.39$ |
| | 30 ng/kg/min | 5 | $79.8 \pm 29.6$ | $+ 1.02^{a} \pm 0.20$ |
| | 100 ng/kg/min | 5 | $64.6 \pm 23.7$ | $+ 1.32^{a} \pm 0.20$ |
| ASS | 10 mg/kg | 5 | $26.1 \pm 13.9$ | $+ 0.06^{n.s.} \pm 0.13$ |
| Cicaprost 10 ng/kg/min + ASS 10 mg/kg | | 5 | $13.8 \pm 6.7$ | $+ 1.27^{a,b} \pm 0.54$ |

[a] signifikant gegen Kontrolle (t-Test, $p < 0.05$)
[b] signifikant gegen ASS/Cicaprost Einzeleingaben ($p < 0.05$)
[n.s.] nicht signifikant

## Beispiel 4

An narkotisierten Meerschweinchen wird ein 2 cm langes Stück der externen linken Karotisarterie sorgfältig freipräpariert und über eine kleine Metallplatte gezogen. Anschließend wird die Gefäßwand über eine Länge von 1 cm für 3 Min mit einem 200 g schweren, auf -15°C gekühlten Stahlstempel beschwert. Auf diese Weise kommt es zu einer massiven lokalen Gefäßwandschädigung an einer großen Arterie und zum Heranwachsen eines roten Thrombus an der geschädigten Stelle. 3 h nach der Schädigung werden das geschädigte Segment und ein gleich langes - Stück der kontralateralen Arterie entnommen, gespült und in Hämoglobin-Test-reagenz eingebracht. 24 h später werden die Hämoglobin-Gehalte beider Segmente bestimmt. Aus der Differenz dieser Hämoglobin-Gehalte wird dann der Netto-Hämoglobin-Gehalt des roten Thrombus als Maß der Thrombusgröße errechnet.

Ergebnisse

ASS 5 mg/kg i.v. hat keinen signifikanten Einfluß auf die Thrombusbildung in der Karotisarterie des Meerschweinchens (Tabelle 4). Cicaprost in der ebenfalls nicht signifikant wirksamen Dosierung von 0,3 ng/kg/min i.v. führt in Kombination mit ASS zu einer signifikanten antithrombotischen Wirkung; der Thrombus-Hämoglobin-Gehalt wird um 85 % gegenüber der Kontrolle von 8,6 µmol (Median) auf 1,3 µmol reduziert.

Tabelle 4: Wirkung von Cicaprost, ASS und einer Kombination von Cicaprost + ASS auf die Thrombusbildung in durch Druck + Kälte vorgeschädigten Karotisarterien des Meerschweinchens (Mediane und Quartile der Thrombus-Hämoglobin[Hb]-Gehalte).

|  | Thrombus Hb-Gehalt [$^4$mol] | | |
|---|---|---|---|
|  | N | Median | Q 25 / Q 75 |
| Kontrolle | 30 | 8,6 | 5,7 / 12,1 |
| Cicaprost 0,3 ng/kg/min | 10 | 12,4[n.s.] | 8,0 / 25,0 |
| 1,0 ng/kg/min | 10 | 1,9[a] | 0,2 / 5,8 |
| 3,0 ng/kg/min | 11 | 0[a] | -0,4 / 0,7 |
| ASS 5 mg/kg | 10 | 3,4[n.s.] | -0,1 / 13,8 |
| Cicaprost 0,3 ng/kg/min + ASS 5 mg/kg | 10 | 1,3[a,b] | -1,2 / 5,1 |

[a] signifikant gegen Kontrolle ( = 5 %, Rangsummentest)
[b] signifikant gegen Cicaprost
[n.s.] nicht signifikant

## Patentansprüche

**Patentansprüche für folgende Vertragstaaten : AT, BE, CH, DE, DK, FR, GB, IT, LI, LU, NL, SE**

1. Kombinationserzeugnis zur Hemmung der Thrombozytenfunktion und zur Thrombosebehandlung, enthaltend die Carbacyclinderivate Cicaprost oder Eptaloprost und Acetylsalicylsäure (ASS).

2. Erzeugnis gemäß Anspruch 1, dadurch gekennzeichnet, daß die Acetylsalicylsäure und das Carbacyclinderivat in getrennten Dosiseinheiten vorliegen.

3. Erzeugnis gemäß Anspruch 1, dadurch gekennzeichnet, daß die Acetylsalicylsäure und das Carbacyclinderivat in einer Dosiseinheit vorliegen.

4. Erzeugnis gemäß Anspruch 1, dadurch gekennzeichnet, daß die Acetylsalicylsäure und das Carbacyclinderivat in einem Gewichtsverhältnis 1:5·10⁻⁶ bis 1:1 zueinander stehen.

5. Erzeugnis gemäß Anspruch 1, enthaltend eine Carbacyclinderivat-Dosiseinheit von 1-1000 µg und eine Acetylsalicylsäure-Dosiseinheit von 1-500 mg.

6. Verwendung von Kombinationserzeugnissen aus Acetylsalicylsäure und den Carbacyclinderivaten Cicaprost oder Eptaloprost in Verbindung mit üblichen Hilfs- und Trägerstoffen zur Herstellung eines Arzneimittels zur Hemmung der Thrombozytenfunktion und zur Behandlung der Thrombose.

**Patentansprüche für folgende Vertragsstaaten: ES, GR**

1. Verfahren zur Herstellung eines Kombinationserzeugnisses zur Hemmung der Thrombozytenfunktion und zur Thrombosebehandlung, dadurch gekennzeichnet, daß man die Carbacyclinderivate Cicaprost oder Eptaloprost und Acetylsalicylsäure mit üblichen Hilfs- und Trägerstoffen in eine geeignete Darreichungsform gebracht werden.

2. Verfahren zur Herstellung eines Kombinationspräparates nach Anspruch 1, dadurch gekennzeichnet, daß die Acetylsalicylsäure und das Carbacyclinderivat in einem Gewichtsverhältnis 1 : 5 x 10⁻⁶ bis 1 : 1 zueinander stehen.

**Claims**

**Claims for the following Contracting States : AT, BE, CH, DE, DK, FR, GB, IT, LU, NL, SE**

1. Combination product for inhibiting thrombocyte function and for treating thrombosis, comprising the carbacyclin derivative Cicaprost or Eptaloprost and acetylsalicylic acid (ASS).

2. Product according to claim 1, characterised in that the acetylsalicylic acid and the carbacyclin derivative are present in separate dosage units.

3. Product according to claim 1, characterised in that the acetylsalicylic acid and the carbacyclin derivative are present in one dosage unit.

4. Product according to claim 1, characterised in that the acetylsalicylic acid and the carbacyclin derivative are in a ratio by weight to one another of 1:5x10⁻⁶ to 1:1.

5. Product according to claim 1, containing a carbacyclin dosage unit of 1-1000 µg and an acetylsalicylic acid dosage unit of 1-500 mg.

6. Use of combination products of acetylsalicylic acid and the carbacyclin derivative Cicaprost or Eptaloprost in combination with customary excipients and carriers for the preparation of a medicament for inhibiting thrombocyte function and for treating thrombosis.

**Claims for the following Contracting States : ES, GR**

1. A process for preparing a combination product for inhibiting thrombocyte function and for treating thrombosis, characterized in that the carbacycline derivative Cicaprost or Eptaloprost and acetylsalicyclic acid (ASS) are combined with customary excipients and carriers.

2. A process according to claim 1, characterized in that the acetylsalicylic acid and the carbacycline derivative are in a ratio by weight to one another of 1 : 5 x 10⁻⁶ to 1 : 1.

**Revendications**

**Revendications pour les Etats contractants suivants : AT, BE, CH, DE, DK, FR, GB, IT, LU, NL, SE**

1. Produit combiné pour l'inhibition de la fonction des thrombocytes et pour le traitement de la thrombose, contenant les dérivés de carbacycline cicaprost ou eptaloprost, et de l'acide acétylsalicylique (AAS).

2. Produit selon la revendication 1, caractérisé en ce que l'acide acétylsalicylique et le dérivé de carbacycline se présentent dans des unités de dosage séparées.

3. Produit selon la revendication 1, caractérisé en ce que l'acide acétylsalicylique et le dérivé de carbacycline se présentent dans une unité de dosage.

4. Produit selon la revendication 1, caractérisé en ce que l'acide acétylsalicylique et le dérivé de carbacycline se situent dans un rapport en poids de $1:5.10^{-6}$ à $1:1$.

5. Produit selon la revendication 1, contenant une unité de dosage de dérivé de carbacycline de 1-1000 µg et une unité de dosage d'acide acétylsalicylique de 1-500 mg.

6. Utilisation de produits combinés d'acide acétylsalicylique et des dérivés de carbacycline cicaprost ou eptaloprost en association avec des substances auxiliaires et des supports classiques pour la préparation d'un médicament pour l'inhibition de la fonction des thrombocytes et pour le traitement de la thrombose.

**Revendications pour les Etats contractants suivants: ES, GR**

1. Procédé de preparation d'un produit combiné pour l'inhibition de la fonction des thrombocytes et pour le traitement de la thrombose, caractérisé en ce qu'on melange les dérives de carbacycline cicaprost ou eptaloprost et de l'acide acetylsalicylique (ASS) avec des substances auxiliaires et des supports classiques.

2. Procédé de preparation d'un produit combiné selon la revendication 1, caracterisé en ce que l'acide acetylsalicylique et le derivé de carbacycline se situent dans un rapport en poids de $1 : 5 \times 10^{-6}$ a $1 : 1$.